(19) Europäisches Patentamt
European Patent Office
Office européen des brevets

(11) Publication number: **0 616 772 A1**

# (12) EUROPEAN PATENT APPLICATION

(21) Application number: **94200800.4**

(51) Int. Cl.5: **A01N 43/56**

(22) Date of filing: **25.03.94**

(30) Priority: **26.03.93 EP 93105005**

(43) Date of publication of application: **28.09.94 Bulletin 94/39**

(84) Designated Contracting States: **AT BE CH DE DK ES FR GB GR IE IT LI LU MC NL PT SE**

(71) Applicant: **SHELL INTERNATIONALE RESEARCH MAATSCHAPPIJ B.V.**
**Carel van Bylandtlaan 30**
**NL-2596 HR Den Haag (NL)**

(72) Inventor: **Newton, Trevor William**
**Friedenstrasse 20**
**D-55270 Schwabenheim (DE)**

## (54) Herbicidal pyrazolidindione compositions.

(57) Herbicidal composition comprising a carrier and/or surface-active agent together with, as active ingredient, a 1-phenyl pyrazolidindione of general formula I-:

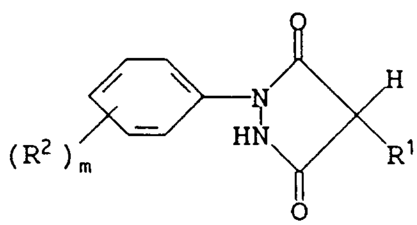

(I)

wherein $R^1$ represents an optionally substituted alkyl, alkenyl, alkynyl, cycloalkyl or aralkyl group;
$R^2$ represents a halogen atom, a formyl, carboxy, azido, nitro or cyano group, an optionally substituted alkoxy, alkylthio, alkenyloxy, alkenylthio, alkynyloxy, alkynylthio, cycloalkoxy, cycloalkylthio, alkylcarbonyl, alkoxycarbonyl, amino, alkylamino or dialkylamino group, or one of the meanings given for $R^1$ ; m represents 0, 1, 2, 3 or 4 and when m is greater than 1 each group $R^2$ may be the same or different;
together with salts of such compounds, and tautomers thereof.

EP 0 616 772 A1

This invention relates to certain 1-herbicidal compositions containing, as active ingredient, 1-phenyl pyrazolidindiones, and their use as herbicides to combat undesired plant growth.

Certain derivatives of pyrazolidin-3,5-dione (and its tautomeric forms) are known to affect plant growth. Thus, European Patent Application 508126 describes 3-hydroxy-4-aryl-5-oxo-pyrazolidine derivatives having alkyl, alkenyl or aryl substituents at the 1-position; such compounds being active against ecto parasites and also certain plant species. European Patent Application 0265305 claims herbicidally active 3-substituted pyrazol-5-ones whose synthesis proceeds via pyrazolidin-3,5-diones having hydrogen or alkyl at the 4-position, these intermediates not being described as possessing any herbicidal activity themselves. PCT patent specification WO 92/16510 describes pyrazolidin-3,5-diones substituted at the 4-position with a phenyl, pyridine or naphthalene group, and at the 1-(and/or 2-) position with an acyclic hydrocarbon group, those compounds being active against insects, mites and certain plant species.

It has now been found that particularly useful herbicidal activity is present in pyrazolidindiones bearing a phenyl substituent at the 1-position and an optionally substituted, non-aromatic hydrocarbon substituent at the 4-position, certain of such compounds being known per se in the above literature.

Accordingly, the present invention provides a herbicidal composition comprising a carrier and/or surface-active agent together with, as active ingredient, a 1-phenyl pyrazolidindione of general formula I-:

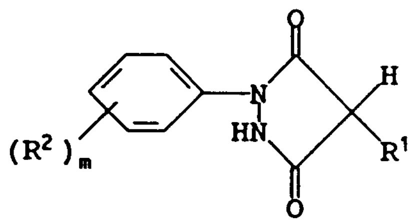

(I)

wherein $R^1$ represents an optionally substituted alkyl, alkenyl, alkynyl, cycloalkyl or aralkyl group;

$R^2$ represents a halogen atom, a formyl, carboxy, azido, nitro or cyano group, an optionally substituted alkoxy, alkylthio, alkenyloxy, alkenylthio, alkynyloxy, alkynylthio, cycloalkoxy, cycloalkylthio, alkylcarbonyl, alkoxycarbonyl, amino, alkylamino or dialkylamino group, or one of the meanings given for $R^1$ ; m represents 0, 1, 2, 3 or 4 and when m is greater than 1 each group $R^2$ may be the same or different;

together with salts of such compounds, and tautomers thereof.

An alkyl, alkenyl or alkynyl radical or moiety may be a straight or branched chain group. Generally an alkyl radical or moiety has from 1 to 12 carbon atoms, preferably from 1 to 6, carbon atoms. Alkenyl and alkynyl radicals or moieties suitably have from 2 to 12 carbon atoms, preferably from 2 to 8, especially from 3 to 5, carbon atoms. Cycloalkyl groups suitably have from 3 to 8, especially 5 or 6, carbon atom ring members.

The aryl moiety in an aralkyl radical may be a single or fused carbocyclic ring system having from 6 to 10 ring members. Suitably an aryl moiety comprises a single ring system and preferably is a phenyl ring.

Certain radicals represented by the symbols $R^1$ and $R^2$ may be unsubstituted or substituted. Where substituents are present, the substituent groups may be any of those customarily employed in the modification and/or development of pesticidal compounds and are especially substituents that maintain or enhance the herbicidal activity associated with the compounds of the present invention, or influence persistence of action, soil or plant penetration, or any other desirable property of such herbicidal compounds. There may be one or more of the same or different substituents present in each radical.

Optional substituents for alkyl, alkenyl, alkynyl, cycloalkyl, alkoxy, alkenyloxy, alkynyloxy, alkylthio, alkenylthio, alkynylthio, alkylamino, alkylcarbonyl or alkoxycarbonyl groups may be independently selected from one or more of halogen atoms and alkoxy, alkenyloxy, hydroxy, alkylthio, alkylsulphonyl, alkylsulphinyl, alkylenedioxy, alkylenedithio, haloalkyl and alkoxycarbonyl groups, and dialkyliminoxy, optionally substituted amino, trialkylsilyl, alkylcarbonyl, alkoxycarbonyl, carboxy, cyano, thiocyanato and optionally substituted aminocarbonyl groups.

Optional substituents for the aryl moiety in an aralkyl group may be independently selected from one or more of halogen atoms and nitro, cyano, alkyl, haloalkyl, alkoxy, alkylthio, aryloxy, alkoxycarbonyl and aralkoxycarbonyl groups.

Suitable examples of group $R^1$ include alkyl groups of up to 6, especially 3 or 4, carbon atoms. Suitable examples of the group $R^2$ include halogen, especially chlorine, atoms, nitro group, and alkyl groups of up to 6 carbon atoms, especially methyl. m is preferably 1 or 2. Higher levels of herbicidal activity tend to be present in the disubstituted compounds (where m = 2), especially when the two substituents are in the 3,5

positions.

The pyrazolidindione may exist in a variety of tautomeric forms, all of which fall within the scope of this invention, together with salts thereof.

The present invention also includes a process for the preparation of compounds of formula I, which comprises reacting a phenyl hydrazine derivative of formula II:-

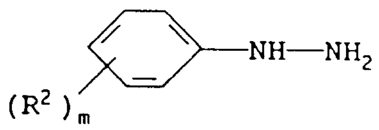

II

with a 2-substituted malonic acid ester of formula III: -

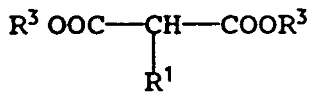

III

wherein $R^1$, $R^2$ and m have the meanings as defined for formula I and $R^3$ represents an alkyl, suitably ethyl, group.

This reaction is suitably carried out by heating together the two reactants under conditions such that the alcohol $R^3OH$ is removed from the reaction mixture by distillation. Suitable conditions include heating at 175° -200°C for several hours. If desired, the reaction may be facilitated, and lower temperatures employed, by the use of a strong base such as sodium ethoxide; under such conditions a convenient reaction temperature is about 100°C.

Alternatively, a malonic acid halide may be used in place of the malonic ester of formula III, the halide having the general formula IV:

$R^1CH(COHal)_2$ ,    IV

wherein $R^1$ is as defined above, and Hal represents a halogen, suitably chlorine, bromine or iodine, atom. When the malonic halide is used, the reaction is preferably initiated below room temperature, suitably 0°C, and is conveniently carried out in a solvent, such as dichloromethane.

The prepared compounds of general formula I may, if desired, be isolated and purified using conventional techniques.

The starting phenyl hydrazines of general formulae II are commercially available, or may be prepared by conventional techniques. Certain of these compounds are novel compounds, and as such form a further aspect of this invention.

Compositions containing compounds of the general formula I have been found to have interesting activity as herbicides having a wide range of pre- and post-emergence activity against undesirable species.

Preferably there are at least two carriers in a composition of the present invention, at least one of which is a surface-active agent.

The present invention additionally encompasses the preparation of such a herbicidal composition by the process of bringing a carrier into association with a compound of the present invention.

The present invention further provides the use of a compound according to the invention as a herbicide.

Further, in accordance with the invention there is provided a method of combating undesired plant growth at a locus by treating the locus with a composition or compound according to the invention. The locus may, for example, be the soil or plants in a crop area. Application to the locus may be pre-emergence or post-emergence. The dosage of active ingredient used may, for example, be from 0.01 to 10kg/ha, preferably 0.01 to 4kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ether; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example, the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The herbicidal composition of the invention may also contain other biologically active ingredients, for example compounds possessing herbicidal, insecticidal or fungicidal properties.

The following Examples illustrate the invention. The structures of the compounds of the invention prepared in the following Examples were confirmed by mass spectrometry and NMR.

## Example 1

### 1-[3,5-Dichlorophenyl],4-n-butylpyrazolidin-3,5-dione

A mixture of diethyl 2-n-butylmalonate (14.4 g, 55 mmol) and 3,5-dichlorophenyl hydrazine (4.8 g, 27 mmol) was stirred and heated at 180-190 °C for 6 hours, during which time ethanol was removed by distillation. The residue was cooled, dissolved in dichloromethane (100 ml) and extracted into 2N sodium hydroxide solution (100 ml). The solution was filtered through active charcoal and acidified with 5N hydrochloric acid. The resulting precipitate was filtered off, washed with water and dried in vacuo to yield the title compound as a white solid, m.pt. 140-142 °C. Analysis Calc. C 51.84; H 4.69; N 9.30%; Found C 51.52; H 4.83; N 9.14%.

## Example 2

### 1-[3,5-Dichlorophenyl],4-s-butylpyrazolidin-3,5-dione

A mixture of diethyl 2-s-butylmalonate (9.0 ml, 40 mmol) and 3,5-dichlorophenylhydrazine (3.5 g, 20 mmol) was stirred and heated at 200 °C for 3 hours and then allowed to cool to 100 °C. A solution of sodium ethoxide, prepared by adding sodium pieces (0.46 g, 20 mg atom) to dry ethanol (30 ml), was added to the stirred mixture at 100 °C and the ethanol removed by distillation. The mixture was then heated for a further 2 hours at 160-180 °C and allowed to cool to room temperature. The residue was dissolved in water and the aqueous solution was washed with diethyl ether and then acidified with glacial acetic acid. The resulting precipitate was filtered off, washed with water and dried in vacuo to yield the title compound as a white solid, m.pt. 117-120 °C. Analysis Calc. C 51.84; H 4.69; N 9.30% Found C 51.42; H 4.79; N

9.14%.

Example 3

1-[4-Chlorophenyl],4-i-butylpyrazolidin-3,5-dione

A solution of 2-i-butylmalonyl chloride (3.9 g, 20 ml) in 1,2-dichloroethane (50 ml) was added dropwise to a stirred solution of pyridine (4.0 ml, 50 mmol) in dichloromethane (100 ml) at -15 ° C. After stirring for 1 hour at this temperature, a solution of 4-chlorophenyl hydrazine (2.9 g, 20 mmol) in 1,2-dichloroethane was added dropwise. The solution was stirred for 1 hour at 0 ° C, 1 hour at room temperature and then 2.5 hours at 60 ° C. After cooling to room temperature, the solution was washed with 5N hydrochloric acid (2 x 50 ml) followed by water (2 x 50 ml). The organic phase was extracted with sodium hydroxide solution (2 x 150 ml) and the aqueous phase was acidified with 5N hydrochloric acid. The resulting precipitate was filtered off, washed with water, dried in vacuo and recrystallised from toluene to yield the title compound as a white solid, m.pt. 175-176 ° C. Analysis Calc C 58.54; H 5.67; N 10.50. Found C 58.21; H 5.77; N 10.00%.

Example 4-14

Following procedures analogous to those described in Examples 1-3, further compounds were prepared, whose details are given in Table I below. In that Table, the compounds are identified by reference to the substituents in the formula I as defined above:

Table 1

| Ex. No. | $R^1$ | m | $R^2$ | M.Pt°C | Analysis (%) Calc/Found C | H | N |
|---|---|---|---|---|---|---|---|
| 4 | isobutyl | 2 | 3,5-$Cl_2$ | 218-220 | 51.84<br>52.13 | 4.69<br>4.74 | 9.30<br>9.36 |
| 5 | isobutyl | 2 | 3,4-$Cl_2$ | 173-174 | 51.84<br>51.98 | 4.69<br>4.66 | 9.30<br>9.06 |
| 6 | isobutyl | 1 | 4-$CH_3$ | 165-166 | 68.27<br>67.91 | 7.37<br>7.21 | 11.37<br>11.20 |
| 7 | isobutyl | 1 | 4-$NO_2$ | 138-139 | 56.31<br>56.50 | 5.45<br>5.55 | 15.15<br>14.61 |
| 8 | isobutyl | 2 | 3,5-$(CH_3)_2$ | 162-163 | 69.21<br>68.74 | 7.74<br>8.02 | 10.76<br>11.35 |
| 9 | n-propyl | 2 | 3,5-$Cl_2$ | 142-143 | 50.20<br>49.65 | 4.21<br>4.42 | 9.76<br>9.74 |
| 10 | isopropyl | 2 | 3,5-$Cl_2$ | 128-129 | 50.20<br>49.97 | 4.21<br>4.44 | 9.76<br>9.64 |
| 11 | isobutyl | 1 | 3-Cl | 112-114 | 58.54<br>58.52 | 5.67<br>5.77 | 10.50<br>10.40 |

Table 1 (cont'd)

| Ex. No. | $R^1$ | m | $R^2$ | M.Pt°C | Analysis (%) Calc/Found C | H | N |
|---|---|---|---|---|---|---|---|
| 12 | isobutyl | 2 | 2,5-$Cl_2$ | oil | Nmr (in $CDCl_3$ solution), ppm: 1.0(6H,d), 1.8(2H,d), 1.9(1H,br. s), 2.1(1H,m) 3.2(1H,t), 7.2-7.5(3H,m) | | |
| 13 | isobutyl | 2 | 2,3-$Cl_2$ | 70 | 51.84<br>50.42 | 4.69<br>4.74 | 9.30<br>8.73 |
| 14 | isobutyl | 1 | 2-Cl | amorph. solid. | Nmr (in $CDCl_3$ solution), ppm: 1.0(6H,d), 1.8(2H,d), 1.9(1H,br. s), 2.1(1H,m), 3.2(1H,t), 7.2-7.6(4H,m) | | |

Example 15

Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissimum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table II below, in which the compounds are identified by reference to the preceding examples. Absence of a numeral in the Table indicates a zero rating, an asterisk indicates that no result was obtained.

TABLE II

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 4 | 4 | 5 | 0 | 0 | 0 | 7 | 7 | 0 | 5 | 2 | 0 | 0 | 0 | 5 | 8 | 8 | 1 | 3 | 5 | 0 | 2 | 5 | 8 | 8 | 0 |
| | | | | | | | | | 1 | 0 | 0 | 0 | 0 | 3 | 6 | 5 | 0 | 0 | 4 | 0 | 0 | 0 | 4 | 3 | 0 |
| 8 | 5 | 4 | 6 | 2 | 0 | 5 | 6 | 0 | 5 | 7 | 6 | 4 | 3 | 6 | 7 | 8 | 2 | 2 | 8 | 2 | 0 | 4 | 7 | 4 | 0 |
| | | | | | | | | | 1 | 4 | 5 | 2 | 0 | 5 | 7 | 7 | 0 | 0 | 2 | 0 | 0 | 0 | 5 | 0 | 0 |
| 9 | 0 | 0 | 0 | 0 | 0 | 0 | 0 | 0 | 5 | 0 | 0 | 0 | 0 | 0 | 7 | 6 | 2 | 0 | 0 | 0 | 0 | 0 | 5 | 0 | 0 |
| | | | | | | | | | 1 | 0 | 0 | 0 | 0 | 0 | 6 | 2 | 0 | 0 | 0 | 0 | 0 | 0 | 0 | 0 | 0 |
| 10 | 0 | 0 | 0 | 0 | 0 | 0 | 0 | 0 | 5 | 1 | 0 | 5 | 1 | 6 | 7 | 8 | 4 | 0 | 0 | 0 | 0 | 0 | 0 | 0 | 0 |
| | | | | | | | | | 1 | 0 | 0 | 0 | 0 | 0 | 3 | 0 | 0 | 0 | 0 | 0 | 0 | 0 | 0 | 0 | 0 |
| 13 | 0 | 0 | 0 | 0 | 0 | 0 | 0 | 0 | 5 | 4 | 0 | 4 | 2 | 2 | 6 | 5 | 2 | 0 | 0 | 0 | 0 | 0 | 0 | 0 | 0 |
| | | | | | | | | | 1 | 2 | 0 | 2 | 0 | 0 | 6 | 2 | 0 | 0 | 0 | 0 | 0 | 0 | 0 | 0 | 0 |

## Claims

1. Herbicidal composition comprising a carrier and/or surface-active agent together with, as active ingredient, a 1-phenyl pyrazolidindione of general formula I-:

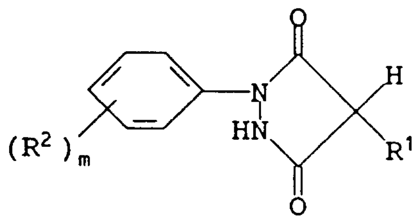

(I)

wherein $R^1$ represents an optionally substituted alkyl, alkenyl, alkynyl, cycloalkyl or aralkyl group; $R^2$ represents a halogen atom, a formyl, carboxy, azido, nitro or cyano group, an optionally substituted alkoxy, alkylthio, alkenyloxy, alkenylthio, alkynyloxy, alkynylthio, cycloalkoxy, cycloalkylthio, alkylcarbonyl, alkoxycarbonyl, amino, alkylamino or dialkylamino group, or one of the meanings given for $R^1$ ; m represents 0, 1, 2, 3 or 4 and when m is greater than 1 each group $R^2$ may be the same or different; together with salts of such compounds, and tautomers thereof.

**2.** Compositions as claimed in claim 1 wherein $R^1$ represents an alkyl group of up to 6 carbon atoms.

**3.** Compositions as claimed in claim 2 wherein $R^1$ represents a propyl or butyl group.

**4.** Compositions as claimed in claim 1, 2 or 3 wherein $R^2$ represents a halogen atom, a nitro group or an alkyl group of up to 6 carbon atoms

**5.** Compositions as claimed in claim 4 wherein $R^2$ represents a chlorine atom or a nitro or methyl group.

**6.** Compositions as claimed in any one of the preceding claims wherein m is 1 or 2.

**7.** Compositions as claimed in claim 6 wherein m is 2 and the two substituents $R^2$ are in the 3 and 5 positions.

**8.** Compositions as claimed in claim 1 wherein the active ingredient is a 1-phenyl pyrazolidindione specifically named herein.

**9.** Method of controlling undesired plant growth at a locus, which comprises treating the locus with a herbicidally effective amount of a composition as claimed in any one of claims 1 to 8.

**10.** Method as claimed in claim 9 wherein the dosage rate of active ingredient applied to the locus is from 0.01 to 10 kg/ha.

European Patent Office

# EUROPEAN SEARCH REPORT

Application Number
EP 94 20 0800

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A,D | EP-A-0 265 305 (ROUSSEL-UCLAF)<br>--- | | A01N43/56 |
| A,D | WO-A-92 16510 (CIBA-GEIGY)<br>----- | | |

| TECHNICAL FIELDS SEARCHED (Int.Cl.5) |
|---|
| A01N<br>C07D |

The present search report has been drawn up for all claims

| Place of search | Date of completion of the search | Examiner |
|---|---|---|
| THE HAGUE | 8 June 1994 | Decorte, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document